# EUROPEAN PATENT SPECIFICATION

(11) **EP 1 987 844 B1**
(45) Date of publication and mention of the grant of the patent: **04.12.2013**
(21) Application number: 07708406.9
(22) Date of filing: 15.02.2007
(51) Int. Cl.: A61K 47/32, A61K 9/70, A61K 31/497, A61K 31/517, A61K 31/5415, A61K 31/55, A61K 31/5513, A61K 47/46, A61P 25/00, A61P 25/24, A61P 43/00

(54) **ADHESIVE PATCH FOR EXTERNAL USE WITH IMPROVED COHESIVE FORCE AND SUSTAINED-RELEASE CHARACTERISTICS**
KLEBEPFLASTER ZUR ÄUSSERLICHEN ANWENDUNG MIT VERBESSERTER KOHÄSIVER KRAFT UND VERZÖGERTER FREISETZUNG
TIMBRE ADHESIF A USAGE EXTERNE AVEC FORCE DE COHESION AMELIOREE ET CARACTERISTIQUES DE LIBERATION PROLONGEE

(30) Priority: 15.02.2006 JP 2006037420
(43) Date of publication of application: 05.11.2008
(73) Proprietor: Hisamitsu Pharmaceutical Co., Inc., Tosu-shi, Saga 841-0017 (JP)
(72) Inventor: UCHIDA, Naoyuki, Tsukuba-shi, Ibaraki 3050856 (JP); KURIBAYASHI, Mitsuru, Tsukuba-shi, Ibaraki 3050856 (JP)
(74) Representative: Adam, Holger
(86) International application number: PCT/JP2007/052676
(87) International publication number: WO 2007/094385

(56) References cited:
- WO-A1-95/18603
- WO-A1-2005/025546
- WO-A1-2005/025546
- WO-A2-2004/039427
- WO-A2-2007/035942
- JP-A- 11 012 164
- JP-B2- 2 977 254
- US-A1- 2002 192 300
- US-A1- 2002 192 300

## Description

### Technical Field

The present invention relates to an adhesive patch for external use for administering a drug, preferably a basic drug and/or a pharmaceutically acceptable salt thereof transdermally into a living body by adhering to the skin of the body.

### Background Art

Generally in the case of transdermally administering a drug, it is preferable that this drug is in a dissolved state in an adhesive, from the viewpoints of the diffusivity of the drug, transferability to the skin, and the like. Moreover, an absorption enhancer is blended in order to secure a predetermined dose of the drug. Therefore, there are many cases where liquid components such as solubilizing agents, solvent promoter, absorption enhancers, and the like, are used in large quantities so as to realize sufficient skin permeability. However, there has been a problem that using these components causes a decrease in the cohesive force of the adhesive base. On the other hand, in general, when the cohesive force is increased, the amount of skin permeation tends to decrease, and thus increasing the cohesive force by using more adhesive leads to a decrease in the amount of permeation. Particularly, in the case of a poorly soluble drug, it is common that the skin permeability is also low, and there is a need to blend large amounts of solubilizing agents or solvent promoter, or further absorption enhancers into transdermal absorption preparations. As a result, a decrease in the cohesiveness of adhesive bases has been a problem.
In other words, there has been a demand for the development of a transdermal absorption preparation which shows good drug skin permeability while having a large quantity of liquid components blended in the adhesive and still maintaining cohesiveness, and can realize the control for constant drug absorption.

For this reason, for example, as the means for enhancing the solubility of a drug in an adhesive, it has been proposed to copolymerize an aminoalkyl (meth)acrylamide having an amino group as a pressure-sensitive adhesive of the adhesive layer (see Patent Document 1). However, to select this copolymer by only considering the solubility of drugs and not taking into account the cohesiveness of the adhesive was not realistic, thus a method could not be said to be practical.
Furthermore, a transdermal absorption adhesive patch characterized in that a drug being in the form of a pharmaceutically acceptable acid salt is contained in the plaster layer in a proportion of 0.1 to 20% by weight, and a polymer containing basic nitrogen and having no adhesiveness to the skin at ambient temperature, for example, a polymerizable amine copolymer such as vinylpyrrolidone, is contained in the plaster layer as a solubility enhancer for drugs, in a proportion of 0.1 to 50% by weight (see Patent Document 2) has also been proposed. Because a solubility enhancer for drugs was used, an improvement of the transdermal absorbability of drugs was shown, however, there was a disadvantage that satisfying cohesiveness cannot be obtained.
A reservoir type preparation for transdermal administration comprised of a hydrophilic acrylate polymer containing a cationic functional group (see Patent Document 3) has also been proposed, but the production method is very complicated.
A sheet-like packed agent comprised of a mixture of a first component including polyvinylpyrrolidone and/or a copolymer including vinylpyrrolidone as a main component, and a second component including a polymer of acrylic acid and/or methacrylic acid, characterized in that the ratio of the content of the vinylpyrrolidone component in the first component and the content of the (meth)acrylic acid component in the second component is 97 : 3 to 70 : 30, has been proposed (see Patent Document 4). However, there is no disclosure at all concerning the transdermal absorbability of drugs, and particularly, poorly soluble drugs.
In this way, an attempt has been made to maintain drugs in a stable manner and to improve transdermal absorption, but it is still not possible to provide a well-balanced, practical transdermal absorption preparation which can maintain cohesiveness while having a large amount of liquid components blended in the adhesive of the adhesive patch, shows good drug skin permeability, and is capable of controlling the drug absorption to be constant.

Meanwhile, risperidone is a benzisoxazole derivative compound developed by Janssen Pharmaceutica NV (Belgium). As for its pharmacological action, anti-dopamine action, anti-serotonin action, and catalepsy inducing action have been confirmed, and at present, the compound is used in a wide range of clinical fields as a therapeutic agent for schizophrenia. The effects of risperidone on schizophrenia are considered to be mainly resulting from the control of the central nervous system based on the dopamine D₂ receptor antagonistic action and serotonin 5-HT₂ receptor antagonistic action. Furthermore, risperidone exhibits excellent effects on positive symptoms such as hallucination and delusion, as well as strong effects on negative symptoms such as emotional withdrawal and blunted affect, and is characterized by having relatively fewer extrapyramidal side effects (tremor, rigor, and the like) compared to conventional typical antipsychotic drugs. Thus, risperidone is considered to be a very useful therapeutic agent for schizophrenia which can significantly improve patients' quality of life (QOL).

As the method of administering risperidone, oral administration methods using tablets, fine granules, internal liquid preparations, and the like have been traditionally used. However, oral administration presents disadvantages such as that the drug is subjected to the first pass effect in the liver after being absorbed, or a blood concentration which is higher than necessary is temporarily observed after administration, or the like. Furthermore, oral administration is frequently reported to be accompanied by side effects such as gastrointestinal distress, nausea and anorexia, and thus about 75% of schizophrenic patients complain of difficulties in regularly taking in the oral preparations of risperidone. Therefore, for the purpose of overcoming such problems in oral administration and providing a preparation which can be easily taken by patients safely and steadily, a method of administering risperidone using an adhesive patch has been investigated in these years. The method of administering risperidone using an adhesive patch can overcome the above-described problems associated with the methods for oral administration, and has advantages such as reduced number of administrations, improved compliance, and ease of administration and cessation, thus being anticipated as a useful method of administration.

Furthermore, Patent Document 5 describes formulating a skin penetration enhancer including a fatty acid or a fatty acid ester for the transdermal administration of risperidone.

Patent Document 1: JP-B No. 7-45400
Patent Document 2: Japanese Patent No. 2977252
Patent Document 3: JP-B No. 6-67835
Patent Document 4: Japanese Patent No. 2968021
Patent Document 5: JP-W No. 11-503138

### Disclosure of the Invention

### Problems to be Solved by the Invention

It is an object of the present invention to obtain a transdermal absorption preparation which shows good drug skin permeability of a poorly soluble drug, while having a large quantity of liquid components blended in the adhesive and still maintaining good formulation properties (cohesiveness, and the like), and can keep an effective blood concentration of a drug at a constant value over a long time.

### Means for Solving the Problems

The inventors of the present invention have carried out development of a well-balanced adhesive layer which can satisfy all of the characteristics of the cohesiveness for an adhesive patch, the skin permeability of a drug (particularly a poorly soluble drug) and sustained drug release, and discovered that when polyvinylpyrrolidone and/or a copolymer including vinylpyrrolidone as a main component, and a (meth)acrylic acid ester-based copolymer containing a basic nitrogen atom and/or a cationic nitrogen atom are contained in the adhesive layer of an adhesive patch, the adhesive patch for external use exhibits ideal sustained release permeation while maintaining good formulation properties (cohesiveness, and the like), and thus completed the invention. Furthermore, the inventors discovered that as the drug, basic drugs such as chlorpromazine hydrochloride, imipramine hydrochloride, risperidone, aripiprazole and olanzapine are preferred.

The present invention relates to an adhesive patch for external use which is a pharmaceutical preparation having an adhesive layer containing a drug provided on a support, characterized in that the adhesive layer contains "polyvinylpyrrolidone and/or a copolymer including vinylpyrrolidone as a main component" and "a (meth)acrylic acid ester-based copolymer containing a basic nitrogen atom and/or a cationic nitrogen atom."
The present invention also relates to an adhesive patch for external use in which the contents of the polyvinylpyrrolidone and/or copolymer including vinylpyrrolidone as a main component, and the content of the (meth)acrylic acid ester-based copolymer containing a basic nitrogen atom and/or a cationic nitrogen atom are 0.1 to 20% by weight and 0.1 to 20% by weight, respectively, based on the total amount of the adhesive layer.
The present invention also relates to an adhesive patch for external use in which the (meth)acrylic acid ester-based copolymer containing a basic nitrogen atom and/or a cationic nitrogen atom is a methyl methacrylate-butyl methacrylate-dimethylaminoethyl methacrylate copolymer, or an ethyl acrylate-methyl methacrylate-trimethylammoniumethyl methacrylate chloride copolymer.

The present invention, when described in more detail, is as follows.
(1) An adhesive patch for external use, being a pharmaceutical preparation having an adhesive layer containing a drug provided on a support, wherein the adhesive layer contains polyvinylpyrrolidone and/or a copolymer including vinylpyrrolidone as a main component, and a (meth)acrylic acid ester-based copolymer containing a basic nitrogen atom and/or a cationic nitrogen atom.
(2) The adhesive patch for external use according to (1) above, wherein the polyvinylpyrrolidone and/or copolymer including vinylpyrrolidone as a main component is polyvinylpyrrolidone.
(3) The adhesive patch for external use according to (1) or (2) above, wherein the content of the polyvinylpyrrolidone and/or copolymer including vinylpyrrolidone as a main component is 0.1 to 20% by weight, and the content of the (meth)acrylic acid ester-based copolymer containing a basic nitrogen atom and/or a cationic nitrogen atom is 0.1 to 20% by weight, based on the total amount of the adhesive layer.
(4) The adhesive patch for external use according to any one of (1) to (3) above, wherein the (meth)acrylic acid ester-based copolymer containing a basic nitrogen atom and/or a cationic nitrogen atom is a methyl methacrylate-butyl methacrylate-dimethylaminoethyl methacrylate copolymer, or an ethyl acrylate-methyl methacrylate-trimethylammoniumethyl methacrylate chloride copolymer.
(5) The adhesive patch for external use according to any one of (1) to (4) above, wherein the mixing ratio of the polyvinylpyrrolidone and/or copolymer including vinylpyrrolidone as a main component, and the (meth)acrylic acid ester-based copolymer containing a basic nitrogen atom and/or a cationic nitrogen atom is 1 : 0.01 to 1 : 1 by mass.
(6) The adhesive patch for external use according to any one of (1) to (5) above, wherein the adhesive base in the adhesive layer is an acrylic adhesive base, a rubber-based adhesive base or a silicone-based adhesive base.
(7) The adhesive patch for external use according to any one of (1) to (6) above, wherein the drug is a basic drug.
(8) The adhesive patch for external use according to (7) above, wherein the basic drug is one or two or more drugs selected from the group consisting of chlorpromazine hydrochloride, imipramine hydrochloride, risperidone, aripiprazole and olanzapine.

### Effects of the Invention

According to the present invention, there is provided a transdermal absorption preparation which has good drug skin permeability of a poorly soluble drug while having a large quantity of liquid components blended in the adhesive and still maintaining good formulation properties (cohesiveness, and the like), and can keep an effective blood concentration at a constant value over a long time.

### Best Mode for Carrying Out the Invention

Hereinafter, suitable exemplary embodiments of the present invention will be described in detail.
The drug used in the transdermal absorption preparation of the present invention is not particularly limited as long as it does not exert particularly adverse influence on the basic nitrogen atom or cationic nitrogen of the component blended in the adhesive. Among them, basic drugs may be preferably used, and the examples thereof include anti-dementia drugs (donepezil hydrochloride, and the like), urination disorder improving drugs (tamsulosin hydrochloride, and the like), soporific/sedative agents (flurazepam hydrochloride, rilmazafone hydrochloride, and the like), antipyretic anti-inflammatory analgesics (butorphanol tartrate, perisoxal citrate, and the like), stimulants/analeptics (methamphetamine hydrochloride, methylphenidate hydrochloride, and the like), psychoneurotic drugs (chlorpromazine hydrochloride, imipramine hydrochloride, risperidone, aripiprazole, olanzapine, and the like), local anesthetics (lidocaine hydrochloride, procaine hydrochloride and the like), drugs for urinary organs (oxybutynin hydrochloride, and the like), skeletal muscle relaxants (tizanidine hydrochloride, eperisone hydrochloride, pridinol mesylate, and the like), autonomic drugs (carpronium chloride, neostigmine bromide and the like), antiparkinsonian drugs (trihexyphenidyl hydrochloride, amantadine hydrochloride, pergolide mesylate, and the like), antihistamine agents (clemastine fumarate, diphenhydramine tannate, and the like), bronchodilators (turobuterol hydrochloride, procaterol hydrochloride and the like), cardiotonic agents (isoprenaline hydrochloride, dopamine hydrochloride, and the like), coronary vasodilators (diltiazem hydrochloride, verapamil hydrochloride, and the like), peripheral vasodilators (nicametate citrate, trazoline hydrochloride, and the like), circulatory drugs (flunarizine hydrochloride, nicardipine hydrochloride, and the like), anti-arrhythmic drugs (propranolol hydrochloride, alprenolol hydrochloride, and the like), anti-allergic agents (ketotifen fumarate, azelastine hydrochloride, and the like), anti-vertigo drug (betahistine mesylate, diphenidol hydrochloride, and the like), serotonin receptor antagonistic antiemetic drugs, and narcotic analgesics (morphine sulfate, fentanyl citrate, and the like). Among these, in particular, chlorpromazine hydrochloride, imipramine hydrochloride, risperidone, aripiprazole, olanzapine, and the like are preferred.
Such a drug may be used in the form of a free base, or may also be used in the form of a pharmaceutically acceptable acid salt. It is also acceptable to use the two forms in combination. Furthermore, the drugs as described above may be used individually alone, or may also be used in combination of two or more kinds. These drugs are preferably blended in a proportion of 3 to 30% by weight, more preferably 5 to 20% by weight, and particularly preferably 10 to 20% by weight, based on the total compositional weight of the transdermal absorption preparation, from the viewpoints of formulation properties and transdermal absorbability.

The adhesive base used in the adhesive patch of the present invention is not particularly limited as long as it can serve as a base of the adhesive layer, and examples thereof include an acrylic adhesive base, a rubber-based adhesive base, a silicone-based adhesive base, and the like.
As for the acrylic adhesive base, a homopolymer or a copolymer of a (meth)acrylic ester and/or a copolymer of the (meth)acrylic acid alkyl ester and other functional monomer is suitably used.
Additionally, in the present specification, the term "(meth)acrylic acid" is used to mean acrylic acid or meth-acrylic acid (methacrylic acid). Therefore, for example, the term (meth)acrylic acid ester means acrylic acid ester or methacrylic acid ester.
As for the rubber-based adhesive base, natural rubber, synthetic rubber, styrene-isoprene-styrene block copolymers (SIS), isoprene rubber, polyisobutylene (PIB), styrene-butadiene-styrene block copolymers (SBS), styrene-butadiene rubber (SBR), polybutene, and the like are preferably used.
As for the silicone-based adhesive base, those containing polydimethylsiloxane as a main component, and the like may be used.
Among the adhesive bases, in particular, SIS and (meth)acrylic ester copolymers are preferred, and more preferably, (meth)acrylic acid ester copolymers may be mentioned.

These adhesive bases may be used individually alone, or may also b used in combination of two or more kinds. Considering the formation of adhesive layer and skin permeability of active ingredients, the amount of the adhesive base to be blended is preferably 10 to 80% by weight, more preferably 20 to 70% by weight, and particularly preferably 30 to 70% by weight, based on the total compositional weight of the adhesive layer.

The polyvinylpyrrolidone and/or copolymer including vinylpyrrolidone as a main component according to the present invention is a copolymer including one or two or more components selected from the group consisting of polyvinylpyrrolidone and copolymers containing vinylpyrrolidone as a main component, and for example, in the case of polyvinylpyrrolidone, there may be mentioned Kollidon (BASF Japan, Ltd.), Plasdone (ISP Japan, Ltd.), Povidone K17 (ISP Japan, Ltd.), and the like, and in the case of the copolymer including vinylpyrrolidone as a main component, there may be mentioned PVP/VA (ISP Japan, Ltd.), Luviskol VA series (BASF Japan, Ltd.), and the like. In order to facilitate cohesion of the adhesive, it is necessary to add the polyvinylpyrrolidone and/or copolymer including vinylpyrrolidone as a main component in an amount of 0.1 % by weight or more based on the entirety of the adhesive layer, and the amount is preferably 0.1 to 30% by weight, and more preferably 0.5 to 20% by weight.

The (meth)acrylic acid ester-based copolymer containing a basic nitrogen atom and/or a cationic nitrogen atom according to the present invention is a copolymer including a (meth)acrylic acid ester containing at least one nitrogen atom selected from the group consisting of a basic nitrogen atom and a cationic nitrogen atom, and a neutral (meth)acrylic acid ester such as methyl ester or ethyl ester, and examples thereof include copolymers of acrylate-methacrylate esters containing a neutral ester group such as methyl ester or ethyl ester and a cationic ester group such as trimethylaminoethyl ester; copolymers including methyl methacrylate, butyl methacrylate and dimethylaminomethyl methacrylate; and the like.
Among these, in particular, a polymer of acrylate and methacrylate esters containing a neutral ester group such as a methyl ester group and an ethyl ester group and a cationic ester group such as a trimethylaminoethyl ester group (Eudragit RS100, registered trademark, Rohm Co., Ltd.), and a copolymer of methyl methacrylate, butyl methacrylate and dimethylaminomethyl methacrylate (Eudragit E100, Eudragit EPO, registered trademarks, Rohm Co., Ltd.) are particularly preferred. The additive amount needs to be 0.1% by weight or more based on the entirety of the adhesive layer so as to secure the cohesive force, and the amount is preferably 0.1 to 30% by weight, and more preferably 0.5 to 20% by weight. In particular, if suitable sustained release effects are expected, the amount should be 2 to 7% by weight.

The mixing ratio of the "polyvinylpyrrolidone and/or copolymer containing vinylpyrrolidone as a main component" and the "(meth)acrylic acid ester-based copolymer containing a basic nitrogen atom and/or a cationic nitrogen atom" according to the present invention is not particularly limited, but preferably, 1 parts by mass to 200 parts by mass, preferably 3 to 100 parts by mass, or 3 to 80 parts by mass of the "(meth)acrylic acid ester-based copolymer containing a basic nitrogen atom and/or a cationic nitrogen atom" is mixed with 100 parts by mass of the "polyvinylpyrrolidone and/or copolymer containing vinylpyrrolidone as a main component." That is, the mixing ratio by mass of the two components may be 1 : 0.01 to 1 : 2, preferably 1 : 0.03 to 1 : 1, or 1 : 0.03 to 1 : 0.8.

Furthermore, as for the adhesive base used in the present invention, conventionally used tackifiers, for example, rosin-based resins [Ester Gum (Arakawa Chemical Industries, Ltd.), Hariester (Harima Chemicals, Inc.), Pentalyn (Eastman Chemical Company), Foral (Eastman Chemical Company)], terpenic resins [YS Resin (Yasuhara Chemical Co., Ltd.), Piccolyte (Loos & Dilworth, Inc.)], petroleum resins [Arkon (Arakawa Chemical Industries, Ltd.), Regalite (Eastman Chemical Company), Piccolastic (Eastman Chemical Company), Escorez (Exxon Mobil Corp.), Wingtack (Goodyear Chemical, Inc.), Quinton (Nippon Zeon Co., Ltd.)], phenolic resins, xylene resins, and the like may be used.
These tackifiers may be used individually alone, or may be used in combination of two or more kinds. Furthermore, considering sufficient adhesive force required from an adhesive patch and skin irritation upon peeling, the amount of the tackifier to be blended based on the total composition of the adhesive layer is preferably 20 to 90% by weight, more preferably 30 to 60% by weight, and particularly preferably 40 to 60% by weight, based on the total compositional weight of the adhesive layer.

The transdermal absorption preparation of the present invention may also be appropriately blended with a transdermal absorption enhancer. The transdermal absorption enhancer that can be used in the present invention may be exemplified by an aliphatic alcohol such as isostearyl alcohol; a fatty acid such as capric acid; a fatty acid derivative such as propylene glycol monolaurate or isopropyl myristate; propylene glycol, polyethylene glycol, lauric acid diethanolamide, or the like, and among these, propylene glycol monolaurate is particularly preferably used. These transdermal absorption enhancers may be used individually alone, or may be used in combination of two or more kinds. Considering sufficient permeability of the active ingredient into the skin as required from a preparation, skin irritation, and the like, the amount of the absorption enhancer to be blended is preferably 1 to 30% by weight, more preferably 3 to 20% by weight, and particularly preferably 5 to 15% by weight, based on the total compositional weight of the transdermal absorption preparation.

Furthermore, for the adhesive patch for external use of the present invention, if necessary, other additives such as an antioxidant, a filler, a crosslinking agent, a preservative, a melting point lowering agent, an ultraviolet absorbent and an emulsifier may be used. Among these additives, examples of preferred additives include, as for the antioxidant, tocopherol and ester derivatives thereof, ascorbic acid, ascorbic acid stearic acid ester, nordihydroguaiaretic acid, dibutylhydroxytoluene (BHT), butylhydroxyanisole, and the like. As for the filler, talc, kaolin, hydrated silica, light anhydrous silicic acid, aluminum hydroxide, calcium carbonate, magnesium carbonate, silicates (for example, aluminum silicate, magnesium silicate, and the like), silicic acid, barium sulfate, calcium sulfate, calcium zincate, zinc oxide, titanium oxide, and the like are desirable. As for the crosslinking agent, thermosetting resins such as amino resins, phenolic resins, epoxy resins, alkyd resins and unsaturated polyesters; isocyanate compounds, block isocyanate compounds, organic crosslinking agents, and inorganic crosslinking agents such as metals or metal compounds are desirable. As for the preservative, disodium edetate, tetrasodium edetate, ethyl paraoxybenzoate, propyl paraoxybenzoate, butyl paraoxybenzoate, and the like are desirable. As for the melting point lowering agent, acetic acid, propionic acid, butyric acid, lactic acid, benzoic acid, and salicylic acid are desirable. As for the ultraviolet absorbent, p-aminobenzoic acid derivatives, anthranilic acid derivative, salicylic acid derivatives, coumarin derivatives, amino acid-based compounds, imidazoline derivatives, pyrimidine derivatives, dioxane derivatives, and the like are desirable. As for the emulsifier or solubilizing agent, POE sorbitan monostearate, POE sorbitan monolaurate, POE hydrogenated castor oil, polysorbate, macrogol, and the like may be mentioned.

Such additives including an antioxidant, a filler, a crosslinking agent, a preservative, an ultraviolet absorbent and an emulsifier, may be blended altogether in an amount of preferably 30% by weight or less, more preferably 20% by weight or less, and particularly preferably 10% by weight or less, based on the total compositional weight of the adhesive layer of the adhesive patch.
The adhesive base in the transdermal absorption preparation of the present invention is a base which contains substantially no water, and here the term "substantially" implies that there is no process for intentionally blending water to the adhesive base during the production processes. However, it is not intended to exclude even the water contained in the raw materials for production, or the water resulting from the absorption of sweat or the like by the adhesive base during the application of the adhesive to the skin.

As for the support constituting the adhesive patch, it is preferable to use an elastic or non-elastic support which can efficiently release the medicinal properties. Specifically, for example, a film or sheet, or a laminate thereof, a porous membrane, a foam, a woven fabric or a non-woven fabric formed of a synthetic resin such as polyethylene, polyethylene terephthalate, polypropylene, polybutadiene, ethylene-vinyl acetate polymer, polyvinyl chloride, polyester, nylon or polyurethane; or paper or the like may be suitably used.

An absorption enhancer and a drug are mixed using a mixer, and then a base and an oxidation preventing agent (antioxidant) are added thereto. Subsequently, if necessary, other components such as a softening agent and a tackifier are mixed in to prepare a mixture for forming an adhesive layer. According to necessity, a solvent such as ethyl acetate, ethanol, toluene or cyclohexane may be used, but it is preferable to distill off any excess solvent before forming the adhesive layer. Then, this mixture is directly spread on a film as a support to form an adhesive layer, or the mixture is spread on a release-treated paper or film to form an adhesive layer; then a support is loaded thereon, and the adhesive layer is press transferred onto the support.

### Brief Description of the Drawings

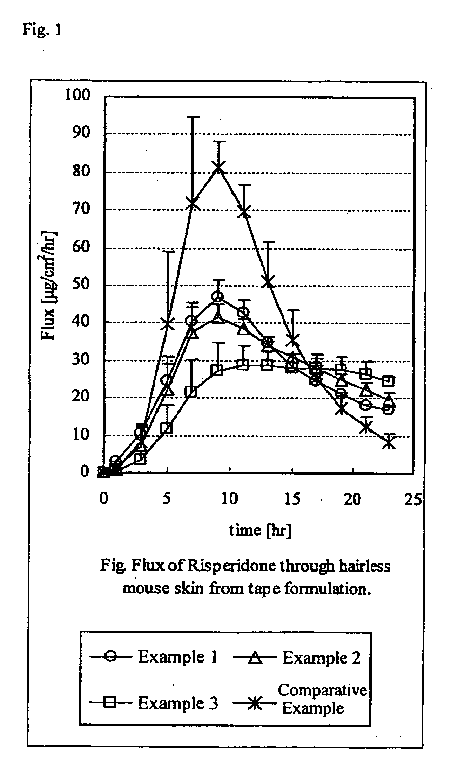

Fig. 1 is a graph showing the results of a test on the amount of drug permeation using hairless mice in the adhesive patch of the present invention and the adhesive patch of Comparative Example.

Hereinafter, the present invention will be described in more detail with reference to Examples. However, the present invention is not intended to be limited to the following Examples.

### EXAMPLE 1

### (Production of adhesive patch)

According to the prescriptions in Table 1 shown below, propylene glycol monolaurate (PGML), risperidone and ethyl acetate were mixed, and then polyvinylpyrrolidone (PVP), Eudragit EPO and an acrylic acid ester copolymer were added thereto. Subsequently, POE sorbitan monostearate, acetic acid, sodium acetate and other base material were blended, and excess solvent was distilled off to prepare a mixture for forming an adhesive layer. Then, this mixture was directly spread on a film as a support to form an adhesive layer, to produce each of the adhesive patches for Examples 1 to 3 and Comparative Example.

**[Table 1]**

| | Example 1 | Example 2 | Example 3 | Comparative Example |
|---|---|---|---|---|
| Acrylic acid ester copolymer | 48.1 | 45.6 | 43.6 | 57.6 |
| Risperidone | 10 | 10 | 10 | 10 |
| PGML | 20 | 20 | 20 | 20 |
| POE sorbitan monostearate | 3 | 3 | 3 | 3 |
| PVP | 10 | 10 | 10 | -- |
| Eudragit EPO | 0.5 | 3 | 5 | 1 |
| Acetic acid | 4.4 | 4.4 | 4.4 | 4.4 |
| Sodium acetate | 4 | 4 | 4 | 4 |
| Thickness (µm) | 82 | 75 | 75 | 81 |

### (Skin permeation test)

The body part skin of a hairless mouse was removed and then was mounted on a flow-through Franz type cell (3.14 cm²) with warm water at 32°C circulating through the external peripheral part, such that the dermis side faced the receptor layer side. Each of the adhesive patches of the present invention produced as described above was adhered on the stratum corneum layer side, and at a rate of 5.5 mL/hr, sampling was performed every two hours up to 24 hours. Physiological saline was used for the receptor layer. The risperidone content in the receptor solution obtained every hour was measured by a high performance liquid chromatography method. The results are presented in Fig 1. The horizontal axis of the graph in Fig. 1 represents time (hour), while the vertical axis represents the amount of drug permeation per hour (µg/cm²/hour). The open circle symbol (○) in the graph of Fig. 1 represents the case of the adhesive patch produced in Example 1; the open triangle symbol (Δ) represents the case of the adhesive patch produced in Example 2; the open square symbol (□) represents the case of the adhesive layer produced in Example 3; and the cross symbol (×) represents the case of the adhesive patch produced in Comparative Example. Each of the vertical lines in the graph of Fig. 1 represents the standard deviation.

It was possible to confirm that the adhesive patches of Examples 1 to 3 all had a good tendency for sustained release compared to the Comparative Example. In particular, it was determined that the adhesive patch of Example 3 exhibited very good sustained release characteristics.

### (Cohesiveness effect)

A preparation produced by the above-described method was cut to an arbitrary size, and peeling of liner from the preparation was performed. The state of peelability was classified into the case where the liner was readily peeled without resistance; the case where peeling of liner was possible, but there was resistance so that marks were left on the plaster surface; the case where peeling of liner was possible, but the plaster was very soft and caused stringiness; and the case where the plaster was too soft so that peeling of liner was impossible, and an evaluation of cohesiveness was performed by a sensory evaluation. The results are presented in the following Table 2.

**[Table 2]**

| | Property evaluation |
|---|---|
| Example 1 | Easy liner peeling, Soft plaster |
| Example 2 | Easy liner peeling |
| Example 3 | Easy liner peeling |
| Comparative Example | Difficult liner peeling |

The adhesive patches of Examples 1 to 3 all showed good cohesiveness compared to the adhesive patch of Comparative Example.

### Industrial Applicability

According to the present invention, there is provided an adhesive patch having risperidone as an active ingredient, which adhesive patch can mildly increase the blood concentration of the drug, as well as has improved compliance so that the effective blood concentration can be maintained at a certain value over a long time. Thus, the invention has high industrial applicability.

## Claims

1. An adhesive patch for external use, being a pharmaceutical preparation having an adhesive layer containing a drug provided on a support, **characterized in that**
the adhesive layer contains polyvinylpyrrolidone and/or a copolymer including vinylpyrrolidone as a main component, and a (meth)acrylic acid ester-based copolymer containing a basic nitrogen atom and/or a cationic nitrogen atom,
wherein the content of the polyvinylpyrrolidone and/or copolymer including vinylpyrrolidone as a main component is 0.1 to 20% by weight, and the content of the (meth)acrylic acid ester-based copolymer containing a basic nitrogen atom and/or a cationic nitrogen atom is 0.1 to 20% by weight, based on the total amount of the adhesive layer.

2. An adhesive patch for external use, being a pharmaceutical preparation having an adhesive layer containing a drug provided on a support, **characterized in that**
the adhesive layer contains polyvinylpyrrolidone and/or a copolymer including vinylpyrrolidone as a main component, and a (meth)acrylic acid ester-based copolymer containing a basic nitrogen atom and/or a cationic nitrogen atom,
wherein the mixing ratio of the polyvinylpyrrolidone and/or copolymer including vinylpyrrolidone as a main component, and the (meth)acrylic acid ester-based copolymer containing a basic nitrogen atom and/or a cationic nitrogen atom is 1 : 0.01 to 1 : 1 by mass.

3. The adhesive patch for external use according to claim 1 or 2, wherein the drug is a basic drug.

4. The adhesive patch for external use according to claim 3, wherein the basic drug is one or two or more drugs selected from the group consisting of chlorpromazine hydrochloride, imipramine hydrochloride, risperidone, aripiprazole and olanzapine.

5. An adhesive patch for external use, being a pharmaceutical preparation having an adhesive layer containing a drug provided on a support, **characterized in that**
the adhesive layer contains polyvinylpyrrolidone and/or a copolymer including vinylpyrrolidone as a main component, and a (meth)acrylic acid ester-based copolymer containing a basic nitrogen atom and/or a cationic nitrogen atom,
wherein the drug is one or two or more drugs selected from the group consisting of chlorpromazine hydrochloride, imipramine hydrochloride, risperidone, aripiprazole and olanzapine.

6. The adhesive patch for external use according to any one of claims 1 to 5, wherein the polyvinylpyrrolidone and/or copolymer including vinylpyrrolidone as a main component is polyvinylpyrrolidone.

7. The adhesive patch for external use according to any one of claims 1 to 6, wherein the (meth)acrylic acid ester-based copolymer containing a basic nitrogen atom and/or a cationic nitrogen atom is a methyl methacrylate-butyl methacrylate-dimethylaminoethyl methacrylate copolymer, or an ethyl acrylate-methyl methacrylate-trimethylammoniumethyl methacrylate chloride copolymer.

8. The adhesive patch for external use according to any one of claims 1 to 7, wherein the adhesive base in the adhesive layer is an acrylic adhesive base, a rubber-based adhesive base or a silicone-based adhesive base.

## Patentansprüche

1. Klebepflaster zur äußerlichen Anwendung, wobei es sich um eine pharmazeutische Zubereitung handelt, die eine Klebschicht aufweist, enthaltend einen Wirkstoff, der auf einem Träger aufgebracht ist, **dadurch gekennzeichnet, dass**
die Klebschicht Polyvinylpyrrolidon und/oder ein Copolymer, enthaltend Vinylpyrrolidon als Hauptkomponente, und ein Copolymer auf (Meth)acrylsäureesterbasis, enthaltend ein basisches Stickstoffatom und/oder ein kationisches Stickstoffatom, enthält,
wobei der Gehalt des Polyvinylpyrrolidons und/oder Copolymers, enthaltend Vinylpyrrolidon als Hauptkomponente, 0,1 bis 20 Gewichtsprozent beträgt und der Gehalt des Copolymers auf (Meth)acrylsäureesterbasis, enthaltend ein basisches Stickstoffatom und/oder ein kationisches Stickstoffatom, 0,1 bis 20 Gewichtsprozent beträgt, bezogen auf die Gesamtmenge der Klebschicht.

2. Klebepflaster zur äußerlichen Anwendung, wobei es sich um eine pharmazeutische Zubereitung handelt, die eine Klebschicht aufweist, enthaltend einen Wirkstoff, der auf einem Träger aufgebracht ist, **dadurch gekennzeichnet, dass**
die Klebschicht Polyvinylpyrrolidon und/oder ein Copolymer, enthaltend Vinylpyrrolidon als Hauptkomponente, und ein Copolymer auf (Meth)acrylsäureesterbasis, enthaltend ein basisches Stickstoffatom und/oder ein kationisches Stickstoffatom, enthält,
wobei das Mischungsverhältnis des Polyvinylpyrrolidons und/oder des Copolymers, enthaltend Vinylpyrrolidon als Hauptkomponente, und des Copolymers auf (Meth) acrylsäureesterbasis, enthaltend ein basisches Stickstoffatom und/oder ein kationisches Stickstoffatom, 1:0,01 bis 1:1, bezogen auf die Masse, beträgt.

3. Klebepflaster zur äußerlichen Anwendung nach Anspruch 1 oder 2, wobei der Wirkstoff ein basischer Wirkstoff ist.

4. Klebepflaster zur äußerlichen Anwendung nach Anspruch 3, wobei der basische Wirkstoff ein oder zwei oder mehr Wirkstoff(e) ist, ausgewählt aus der Gruppe, bestehend aus Chlorpromazinhydrochlorid, Imipraminhydrochlorid, Risperidon, Aripiprazol und Olanzapin.

5. Klebepflaster zur äußerlichen Anwendung, wobei es sich um eine pharmazeutische Zubereitung handelt, die eine Klebschicht aufweist, enthaltend einen Wirkstoff, der auf einem Träger aufgebracht ist, **dadurch gekennzeichnet, dass**
die Klebschicht Polyvinylpyrrolidon und/oder ein Copolymer, enthaltend Vinylpyrrolidon als Hauptkomponente, und ein Copolymer auf Methacrylsäureesterbasis, enthaltend ein basisches Stickstoffatom und/oder ein kationisches Stickstoffatom, enthält,
wobei der Wirkstoff ein oder zwei oder mehr Wirkstoff(e) ist, ausgewählt aus der Gruppe, bestehend aus Chlorpromazinhydrochlorid, Imipraminhydrochlorid, Risperidon, Aripiprazol und Olanzapin.

6. Klebepflaster zur äußerlichen Anwendung nach einem der Ansprüche 1 bis 5, wobei das Polyvinylpyrrolidon und/oder Copolymer, enthaltend Vinylpyrrolidon als Hauptkomponente, Polyvinylpyrrolidon ist.

7. Klebepflaster zur äußerlichen Anwendung nach einem der Ansprüche 1 bis 6, wobei das Copolymer auf (Meth)acrylsäureesterbasis, enthaltend ein basisches Stickstoffatom und/oder ein kationisches Stickstoffatom, ein Methylmethacrylat-Butylmethacrylat-Dimethylaminoethylmethacrylat-Copolymer oder ein Ethylacrylat-Methylmethacrylat-Trimethylammoniumethylmethacrylatchlorid-Copolymer ist.

8. Klebepflaster zur äußerlichen Anwendung nach einem der Ansprüche 1 bis 7, wobei die Klebstoffbasis in der Klebeschicht eine acrylische Klebstoffbasis, eine kautschukbasierte Klebstoffbasis oder eine silikonbasierte Klebstoffbasis ist.

## Revendications

1. Timbre adhésif pour un usage externe, qui est une préparation pharmaceutique ayant une couche adhésive contenant un médicament fourni sur un support, **caractérisée en ce que**
la couche adhésive contient de la polyvinylpyrrolidone et/ou un copolymère contenant de la vinylpyrrolidone comme composant principal, et un copolymère à base d'ester d'acide (méth)acrylique contenant un atome d'azote basique et/ou un atome d'azote cationique,
dans lequel la teneur en polyvinylpyrrolidone et/ou en copolymère comprenant de la vinylpyrrolidone comme composant principal est de 0,1 à 20 % en poids, et la teneur en copolymère à base d'ester d'acide (méth)acrylique contenant un atome d'azote basique et/ou un atome d'azote cationique est de 0,1 à 20 % en poids, en se basant sur la quantité totale de la couche adhésive.

2. Timbre adhésif pour un usage externe, qui est une préparation pharmaceutique ayant une couche adhésive contenant un médicament fourni sur un support, **caractérisée en ce que**
la couche adhésive contient de la polyvinylpyrrolidone et/ou un copolymère contenant de la vinylpyrrolidone comme composant principal, et un copolymère à base d'ester d'acide (méth)acrylique contenant un atome d'azote basique et/ou un atome d'azote cationique,
dans lequel le rapport de mélange de la polyvinylpyrrolidone et/ou d'un copolymère comprenant de la vinylpyrrolidone comme composant principal, et du copolymère à base d'ester d'acide (méth)acrylique contenant un atome d'azote basique et/ou un atome d'azote cationique est de 1:0,01 à 1:1 en poids.

3. Timbre adhésif pour un usage externe selon la revendication 1 ou 2, dans lequel le médicament est un médicament basique.

4. Timbre adhésif pour un usage externe selon la revendication 3, dans lequel le médicament basique est un ou deux médicaments ou plus choisis dans le groupe constitué par le chlorhydrate de chlorpromazine, le chlorhydrate d'imipramine, la rispéridone, l'aripiprazole et l'olanzapine.

5. Timbre adhésif pour un usage externe, qui est une préparation pharmaceutique ayant une couche adhésive contenant un médicament fourni sur un support, **caractérisée en ce que**
la couche adhésive contient de la polyvinylpyrrolidone et/ou un copolymère contenant de la vinylpyrrolidone comme composant principal, et un copolymère à base d'ester d'acide (méth)acrylique contenant un atome d'azote basique et/ou un atome d'azote cationique,
dans lequel le médicament est un ou deux médicaments ou plus choisis dans le groupe constitué par le chlorhydrate de chlorpromazine, le chlorhydrate d'imipramine, la rispéridone, l'aripiprazole et l'olanzapine.

6. Timbre adhésif pour un usage externe selon l'une quelconque des revendications 1 à 5, dans lequel la polyvinylpyrrolidone et/ou le copolymère contenant de la vinylpyrrolidone comme composant principal est la polyvinylpyrrolidone.

7. Timbre adhésif pour un usage externe selon l'une quelconque des revendications 1 à 6, dans lequel le copolymère à base d'ester d'acide (méth)acrylique contenant un atome d'azote basique et/ou un atome d'azote cationique est un copolymère de méthacrylate de méthyle-méthacrylate de butyleméthacrylate de diméthylaminoéthyle ou un copolymère d'acrylate d'éthyle-méthacrylate de méthyle-chlorure de méthacrylate de triméthylammoniumméthyle.

8. Timbre adhésif pour un usage externe selon l'une quelconque des revendications 1 à 7, dans lequel la base adhésive dans la couche adhésive est une base adhésive acrylique, une base adhésive à base de caoutchouc ou une base adhésive à base de silicone.
